**Europäisches Patentamt**

**European Patent Office**

**Office européen des brevets**

(11) Veröffentlichungsnummer: **0 089 008**
**B1**

(12) **EUROPÄISCHE PATENTSCHRIFT**

(45) Veröffentlichungstag der Patentschrift: **08.08.90**

(21) Anmeldenummer: **83102338.7**

(22) Anmeldetag: **10.03.83**

(51) Int. Cl.⁵: **G 01 N 33/531,**
**G 01 N 33/543**

(54) Hochspezifisches Antiserum gegen Prokollagen-Peptid Col 1 (Typ III) und Prokollagen-Peptid (Typ III), seine Herstellung und Verwendung.

(30) Priorität: **13.03.82 DE 3209149**

(43) Veröffentlichungstag der Anmeldung:
**21.09.83 Patentblatt 83/38**

(45) Bekanntmachung des Hinweises auf die Patenterteilung:
**08.08.90 Patentblatt 90/32**

(84) Benannte Vertragsstaaten:
**AT BE CH DE FR GB IT LI LU NL SE**

(56) Entgegenhaltungen:
**EP-A-0 004 940**

**CHEMICAL ABSTRACTS, Band 90, Nr. 3, 15. Januar 1979, Seite 205, Spalte 1, Zusammenfassung 17888b, Columbus, Ohio, US; P. BRUCKNER et al.: "Three conformationally distinct domains in the amino-terminal segment of type III procollagen and its rapid triple helix = coil transition", & EUR. J. BIOCHEM. 1978, 90(3), 595-603 (Kat. D)**

(73) Patentinhaber: **HOECHST AKTIENGESELLSCHAFT**
**Postfach 80 03 20**
**D-6230 Frankfurt am Main 80 (DE)**

(72) Erfinder: **Timpl, Rupert, Dr.**
**Josef-Haerlinstrasse 3**
**D-8035 Gauting (DE)**
Erfinder: **Brocks, Dietrich, Dr.**
**Buchenstrasse 1**
**D-6257 Hünfelden (DE)**
Erfinder: **Neubauer, Horst, Dr.**
**Am Eichkopf 12**
**D-6240 Königstein/Taunus (DE)**
Erfinder: **Strecker, Helmut, Dr.**
**Odenwaldstrasse 56**
**D-6102 Pfungstadt (DE)**

**Beschreibung**

Die Erfindung betrifft ein Verfahren zur gemeinsamen immunologischen Bestimmung von Prokollagen-Peptid (Typ III) und Prokollagen-Peptid Col 1 (Typ III) und Verfahren zur Herstellung von Anti-Prokollagen-Peptid Col 1 (Typ III)-Serum.

Prokollagen (Typ III) ist eine biosynthetische Vorläuferform eines speziellen Kollagens (Typ III), das hauptsächlich im retikulären Bindegewebe vorkommt. Sie unterscheidet sich von diesem Kollagen (Typ III) durch ein zusätzlich am Aminoende lokalisiertes Peptidsegment (Prokollagen-Peptid (Typ III)), das sich durch Behandlung mit Kollagenase vom Molekül abspalten läßt. Prokollagen-Peptid (Typ III) seinerseits läßt sich mit Kollagenase weiter zu Bruchstücken Col 1, Col 2 und Col 3 spalten, welche mit Hilfe von an sich bekannten proteinchemischen Methoden isoliert werden können (Nowack, H. et al., Eur. J. Biochem. *70*, 205—216 (1976), Bruckner, P. et al., Eur. J. Biochem. *90*, 595—603 (1978)).

Untersuchungen über die Konzentration von Prokollagen-Peptid (Typ III) im Serum zeigten, daß diese Konzentration bei fibrotischen Erkrankungen der Leber erhöht ist (Rhode, H. et al., Eur. J. Clin. Invest. *9*, 451—459 (1979)). Ein radioimmunologisches Verfahren zum Nachweis dieses Antigens ist in der europäischen Patentschrift Nr. 4940 beschrieben. Es ist bekannt, daß durch Immunisierung von Kaninchen mit Prokollagen (Typ III) oder Prokollagen-Peptid (Typ III) ein Antikörper erhalten wird, der eine hohe Affinität gegenüber Prokollagen-Peptid (Typ III) aufweist. Die gegenüber Prokollagen-Peptid Col 1 (Typ III) gefundene Affinität war jedoch gering (Rhode et al., loc. cit.).

Überraschenderweise wurde nun gefunden, daß durch Immunisierung mit Prokollagen-Peptid Col 1 (Typ III) Antikörper erhalten werden, die gegenüber Prokollagen-Peptid Col 1 (Typ III) und Prokollagen-Peptid (Typ III) gleiche Affinität zeigen.

Mit der bisherigen Bestimmungsmethode war es möglich, Prokollagen-Peptid (Type III) im Serum zu erfassen. Die besonderen Eingenschaften der neuen Antikörper gestatten es nun, die Gesamtmenge der beiden Antigene Prokollagen-Peptid (Typ III) und Prokollagen-Peptid Col 1 (Typ III) in Körperflüssigkeiten mittels eines immunologischen Verfahrens zu bestimmen. Da das Abbauprodukt Col 1 von diesen Antikörpern ebenfalls gebunden wird, ermöglicht damit die Messung der Summe von Prokollagen-Peptid (Typ III) und Prokollagen-Peptid Col 1 (Typ III) ein zusätzliche diagnostische Aussage. Dies bedeutet eine wertvolle Erweiterung, besonders bei Krankheiten, die mit erhöhten proteolytischen Enzymaktivitäten im Serum oder im Gewebe verbunden sind. Denn in solchen Fällen ist im Serum das Abbauprodukt Col 1 in hoher Menge zu finden, was jedoch mit den bisherigen Methoden nicht erfaßbar ist.

Die Erfindung betrifft somit:

1. Ein hochspezifisches Antiserum gegen die beiden Antigene Prokollagen-Peptid Col 1 (Typ III) und Prokollagen-Peptid (Typ III),

2. ein Verfahren zur Herstellung des hochspezifischen Antiserums gegen die beiden Antigene Prokollagen-Peptid Col 1 (Typ III) und Prokollagen-Peptid (Typ III), das dadurch gekennzeichnet ist, daß Versuchstiere mit Prokollagen-Peptid Col 1 (Typ III) immunisiert werden und ihr Serum gewonnen wird und

3. die Verwendung der unter 1. charakterisierten Antikörper, zur gemeinsamen immunologischen Bestimmung der beiden Antigene, die dadurch gekennzeichnet ist, daß man entweder

a) eine bestimmte, in Bezug auf die zu untersuchende Probe überschüssige Menge des genannten hochspezifischen Antiserums mit der die genannten Antigene enthaltenden Probe und einer bestimmten Menge eines der beiden Antigene in markierter Form zur Reaktion bringt, den gebildeten Antigen-Antikörper-Komplex abtrennt und die Menge der Markierung im Komplex und/oder Überstand bestimmt, oder

b) eine bestimmte, in Bezug auf die zu untersuchende Probe überschüssige Menge des genannten Antiserums mit der zu untersuchenden Probe umsetzt, die nicht umgesetzte Menge der Antikörper mit einem definierten Überschuß eines der genannten Antigene, die an einen Träger gebunden sind, fixiert und mit einem definierten Überschuß eines zweiten markierten Antikörpers umsetzt und dann die Menge der Markierung des gebundenen und/oder freien zweiten Antikörpers bestimmt.

Es können bei dieser verwendung sowohl die bekannten Radio-Immun-Assay-(RIA)-Varianten als auch die Enzym-Immun-Assay-Varianten und analoge Bestimmungen unter Verwendung andersartiger Markierungen, beispielsweise Fluoreszenzmarkierung, Farbstoffmarkierung und dergleichen angewendet werden. Bei der Verfahrensvariante a) ist die radioaktive Markierung, bei der Variante b) die Enzymmarkierung bevorzugt. Derartige Methoden sind dem Fachmann bekannt und sollen hier nicht im einzelnen aufgeführt werden. Bei diesen Nachweisreaktionen konkurrieren die markierten Prokollagen-Peptide in der aus der europäischen Patentschrift Nr. 4940 an sich bekannten Weise um den Antikörper, so daß im gebildeten Antigen-Antikörper-Komplex die Menge markierten Antigens umso geringer ist, je mehr nicht markierte Antigene in der zu bestimmenden Probe enthalten sind. Deshalb kann entweder die Markierung des Komplexes, beispielsweise die Radioaktivität oder die Enzymaktivität, oder die Markierung des Überstandes nach Abtrennen des Antigen-Antikörper-Komplexes verwendet werden, um anhand einer Eichkurve, die mittels Proben bekannten Gehaltes an Prokollagen-Peptid (Typ III) oder Prokollagen-Peptid Col 1 (Typ III) erstellt wurde, die in der zu untersuchenden Probe enthaltenen Menge Antigen festzustellen. Bei der Verfahrensvariante b) wird—wie auch bei Variante a)—bevorzugt die Markierung

2

im Komplex bestimmt.

Die Trennung des Antigen-Antikörper-Komplexes von der Lösung kann nach den hierfür dem Fachmann bekannten üblichen Methoden erfolgen, wie Abfiltrieren, Absaugen, Abzentrifugieren und dergleichen. Auch ist es möglich, das Antiserum an einen festen Träger, beispielsweise die Innenwand eines Reagenzglases, gebunden einzusetzen.

Vorzugsweise wird das Verfahren so durchgeführt, daß die Trennung des mit hochspezifischem Anti-Prokollagen-Peptid Col 1 (Typ III)-Serum Gebildeten Antigen-Antikörper-Komplexes von dem nicht umgesetzten Antigen durch die Verwendung eines gegen das hochspezifische Serum gerichteten zweiten Antikörpers erfolgt. Bevorzugt wird hier ein Antikörper gegen γ-Immunglobulin der für die Gewinnung des Antiserums verwendeten Tierart. Die Markierung des Antigens, d.h. Prokollagen-Peptids (Typ III) oder Prokollagen-Peptids Col 1 (Typ III), kann mit den für die Markierung von Proteinen bekannten Methoden durchgeführt werden. Im Falle der radioaktiven Markierung mit einem Radionuklid wird vorzugsweise [125]Jod verwendet. Zur Markierung mit diesem Radionuklid wird die Chloramin T-Methode (McConahey, P. J. et al., Int. Arch. Allergy 29 (1966) 185) bevorzugt.

Die Enzym-Immun-Assay-Variante zur Bestimmung von Prokollagen-Peptid (Typ III) und Prokollagen-Peptid Col 1 (Typ III) kann auch dergestalt durchgeführt werden, daß der Antikörper gegen γ-Immunglobulin der betreffenden Tierart, z. B. Vom Kaninchen enzymmarkiert ist. Bei dieser Art des Enzym-Immun-Assay wird der nach Bildung des Antigen-Antikörper-Komplexes verbleibende Teil des Anti-Prokollagen-Peptid-Serums bestimmt, indem man diesen an trägergebundenes Prokollagen-Peptid (Typ III) oder Prokollagen-Peptid Col 1 (Typ III) bindet und anschließend mit enzymmarkiertem Antikörper gegen γ-Immunglobulin des Kaninchens zur Reaktion bringt.

Die Menge gebundenen enzymmarkierten Antikörpers wird anschließend durch Messung der Enzymreaktion bestimmt und ist der unbekannten Menge Prokollagen-Peptid (Typ III) und Prokollagen-Peptid Col 1 (Typ III) in der Probe direkt proportional.

Für die erfindungsgemäße Bestimmungsmethode ist es entscheidend, daß ein geeignetes Antiserum gegen Prokollagen-Peptid Col 1 (Typ III) zur Verfügung steht. Dieses ist dann auch gegen Prokollagen-Peptid (Typ III) affin.

Zur Immunisierung wird zweckmäßigerweise Prokollagen-Peptid Col 1 (Typ III) verwendet. Die Immunisierung kann durch subkutane Injektion von Prokollagen-Peptid Col 1 (Typ III) in Versuchstiere wie Ziegen, vorzugsweise Kaninchen, in Gegenwart des kompletten Freundschen Adjuvans erfolgen. In diesem Fall beträgt die Antigendosis 0,2—0,5 mg pro Tier.

Die folgenden Beispiele erläutern die Erfindung weiter.

Beispiel 1

Herstellung von Prokollagen-Peptid Col 1 (Typ III)

60 mg von Prokollagen-Peptid (Typ III) werden in der erforderlichen Menge eines Puffers (Puffer 1: 0,05 M Tris/HCl, pH 8,0, 0,02 M CaCl$_2$) gelöst und auf 55°C erwärmt. Nach Zugabe von 420 Einheiten Kollagenase wird die Mischung 15 min bei 55°C inkubiert. Nach Abkühlen auf 37°C und Zugabe von 420 Einheiten Kollagenase wird die Inkubation für 3 Std. fortgesetzt. Anschließend wird die Mischung gegen zwei (Puffer (Puffer 2: 0,005 M Tris/HCl, pH 8,6, 2 M Harnstoff; Puffer 3: 0,005 M Tris/HCl, pH 8,6, 8 M Harnstoff) dialysiert und über eine DEAE-Cellulosesäule (1,6×5 cm) gegeben, die mit Puffer 3 äquilibriert war.

Die auf der Säule gebundenen Proteine werden mit einem NaCl-Gradienten (0—0,3 M) eluiert. Das Eluat wird bezüglich der Absorption bei 230 nm und der Antigenaktivität durch Verwendung von Antikörpern überprüft, die spezifisch für Prokollagen-Peptid (Typ III) sind. Der letzte Peak, der aus der Säule eluiert wird, enthält normalerweise Prokollagen-Peptid Col 1 (Typ III). Das Peptid wird durch Dialyse gegen 0,01 M (NH$_4$)$_2$CO$_3$ entsalzt und lyophilisiert.

Durchführung der immunologischen Bestimmung (RIA)

25 µg Prokollagen-Peptid (Typ III) werden mit 1 Milli-Curie Jod 125 nach der Chloramin T-Methode markiert und ungebundenes Jod durch Dialyse entfernt. Die weiteren Schritte bei der Durchführung des Radioimmun-Tests werden vorzugsweise in Gegenwart von 0,04% eines nichtionischen Detergens, wie [R]Tween 20, durchgeführt. Bindungskurven werden mit jeweils 2 ng markiertem Prokollagen-Peptid (Typ III) oder Prokollagen-Peptid Col 1 (Typ III) bestimmt. Die Konzentration der Gesamtmenge an Prokollagen-Peptid (Typ III) und Prokollagen-Peptid Col 1 (Typ III) in einer unbekannten Probe von Serum oder anderen Körperflüssigkeiten wird in folgendem Inhibitionstest bestimmt.

Eine bestimmte Menge des Antikörpers wird mit der unbekannten Probe 16 Std. bei 4°C vorinkubiert und nach Zugabe von 2 ng markierten Peptids weitere 8 Stunden bei 4°C inkubiert. Danach wird ein Überschuß von Antikörpern gegen Kaninchen-Immunglobin zugesetzt und das im Immunkomplex gebundene Antigen aus der Lösung abgetrennt. Die Inhibitionsaktivität der unbekannten Probe wird mit der Aktivität von Standard-Konzentrationen von nichtmarkiertem Prokollagen-Peptid (Typ III) oder Prokollagen-Peptid Col 1 (Typ III) verglichen.

Setzt man als Probe Serum von gesunden Patienten in den Inhibitionstest ein, so findet man typischerweise etwa 3fach so hohe Werte für Prokollagen-Peptid Col 1 (Typ III) plus Prokollagen-Peptid (Typ III) als mit einem RIA für Prokollagen-Peptid (Typ III) gefunden wurde.

Beispiel 2
Immunologische Bestimmung mittels Enzym-Immun-Assay

Die Konzentration der Prokollagen-Peptide (Typ III) in einer unbekannten Probe von Serum oder anderen Körperflüssigkeiten wird mit folgendem Enzym-Immun-Test bestimmt.

Eine bestimmte Menge des Anti-Prokollagen-Peptid-Col 1 (Typ III)-Serums wird mit der unbekannten Probe 2,5 Std. Bei 4°C vorinkubiert. Die Mischung wird dann in eine mit 20 ng Prokollagen-Peptid beschichtete Vertiefung einer Mikrotiterplatte pipettiert und 18 Std. bei 4°C inkubiert. Nach Abgießen des Überstandes und Waschen der Vertiefungen mit physiol. NaCl-Lösung wird eine bestimmte Menge enzymmarkierten Antikörpers (z.B. Peroxidase-markiert) gegen γ-Immunglobulin von Kaninchen zugegeben und 5 Std. bei Raumtemperatur inkubiert. Nach Abgießen des Überstandes und Waschen mit physiol. NaCl-Lösung wird durch Zugabe von Enzymsubstrat (bei Peroxidase-Markierung $H_2O_2$) und Chromogen (z.B. o-Phenylendiamin) die Enzymreaktion gestartet. Nach 30 min Inkubation bei Raumtemperatur wird die Reaktion durch Zugabe von 50 µl 6 M HCl gestoppt. Mit einem geeigneten Photometer wird nach 30 min die Farbintensität der Lösung bestimmt. Diese Farbintensität ist direkt proportional der Menge gebundenen enzymmarkierten Antikörpers und damit der unbekannten Menge an Prokollagen-Peptiden in der Probe. Mit Hilfe einer Eichkurve, die mit Lösungen bekannten Gehalts an Prokollagen-Peptid (Typ III) oder Prokollagen-Peptid Col 1 (Typ III) erstellt wurde, kann dann die Menge Prokollagen-Peptid (Typ III) plus Prokollagen-Peptid Col 1 (Typ III) in der Probe ermittelt werden.

**Patentansprüche für die benannten Vertragsstaaten: BE, CH, DE, FR, GB, IT, LI, LU, NL, SE**

1. Hochspezifisches Antiserum gegen die beiden Antigene Prokollagen-Peptid Col 1 (Typ III) und Prokollagen-Peptid (Typ III), erhältlich durch Immunisierung von Versuchstieren mit Prokollagen-Peptid Col 1 (Typ III).

2. Verfahren zur Herstellung eines hochspezifischen Antiserums gegen die beiden Antigene Prokollagen-Peptid Col 1 (Typ III) und Prokollagen-Peptid (Typ III), dadurch gekennzeichnet daß Versuchstiere mit Prokollagen-Peptid Col 1 (Typ III) immunisiert werden und ihr Serum gewonnen wird.

3. Verwendung der Antikörper des Antiserums nach Anspruch 1 zur gemeinsamen immunologischen Bestimmung der beiden Antigene Prokollagen-Peptid (Typ III) und Prokollagen-Peptid Col 1 (Typ III), dadurch gekennzeichnet, daß man entweder

a) eine bestimmte, in Bezug auf die zu untersuchende Probe überschüssige Menge des genannten hochspezifischen Antiserums mit der die genannten Antigene enthaltenen Probe und einer bestimmten Menge eines der beiden Antigene in markierter Form zur Reaktion bringt, den gebildeten Antigen-Antikörper-Komplex abtrennt und die Menge der Markierung im Komplex und/oder Überstand bestimmt, oder

b) eine bestimmte, in Bezug auf die zu untersuchende Probe überschüssige Menge des genannten Antiserums mit der zu untersuchenden Probe umsetzt, die nicht umgesetzte Menge der Antikörper mit einem definierten Überschuß eines der genannten Antigene, die an einen Träger gebunden sind, fixiert und mit einem definierten Überschuß eines zweiten markierten Antikörper umsetzt und dann die Menge der Markierung des gebundenen und/oder freien zweiten Antikörpers bestimmt.

4. Verwendung nach Anspruch 3, dadurch gekennzeichnet, daß die Markierung eine radioaktive, Enzym- oder Fluoreszenzmarkierung ist.

5. Verwendung nach einem oder mehreren der vorhergehenden Ansprüche, dadurch gekennzeichnet, daß bei der Variante a) die Abtrennung des Antigen-Antikörper-Komplexes mit einem zweiten, gegen die Antikörper affinen Antikörper erfolgt.

6. Verwendung nach einem oder mehreren der vorhergehenden Ansprüche, dadurch gekennzeichnet, daß bei der Variante a) eine radioaktive Markierung eingesetzt wird.

7. Verwendung nach einem oder mehreren der vorhergehenden Ansprüche, dadurch gekennzeichnet, daß die Markierung mit Jod 125 erfolgt.

8. Verwendung nach einem oder mehreren der vorhergehenden Ansprüche, dadurch gekennzeichnet, daß bei der Variante b) ein Enzym zur Markierung dient.

9. Verwendung nach einem oder mehreren der vorhergehenden Ansprüche, dadurch gekennzeichnet, daß die Markierung im Komplex bestimmt wird.

**Patentansprüche den benannten Vertragsstaat: AT**

1. Verfahren zur Herstellung eines hochspezifischen Antiserums gegen die beiden Antigene Prokollagen-Peptid Col 1 (Typ III) und Prokollagen-Peptid (Typ III), dadurch gekennzeichnet, daß Versuchstiere mit Prokollagen-Peptid Col 1 (Typ III) immunisiert werden und ihr Serum gewonnen wird.

2. Verwendung der Antikörper des Antiserums nach Anspruch 1 zur gemeinsamen immunologischen Bestimmung der beiden Antigene Prokollagen-Peptid (Typ III) und Prokollagen-Peptid Col 1 (Typ III), dadurch gekennzeichnet, daß man entweder

a) eine bestimmte, in Bezug auf die zu untersuchende Probe überschüssige Menge des genannten hochspezifischen Antiserums mit der die genannten Antigene enthaltenden Probe und einer bestimmten Menge eines der beiden Antigene in markierter Form zur Reaktion bringt, den gebildeten Antigen-Antikörper-Komplex abtrennt und die Menge der Markierung im Komplex und/oder Überstand bestimmt, oder

b) eine bestimmte, in Bezug auf die zu untersuchende Probe überschüssige Menge des genannten Antiserums mit der zu untersuchenden Probe umsetzt, die nicht umgesetzte Menge der Antikörper mit einem definierten Überschuß eines der genannten Antigene, die an einen Träger gebunden sind, fixiert und mit einem definierten Überschuß eines zweiten markierten Antikörper umsetzt und dann die Menge der Markierung des gebundenen und/oder freien zweiten Antikörpers bestimmt.

3. Verwendung nach Anspruch 2, dadurch gekennzeichnet, daß die Markierung eine radioaktive, Enzym- oder Fluoreszenzmarkierung ist.

4. Verwendung nach einem oder mehreren der vorhergehenden Ansprüche, dadurch gekennzeichnet, daß bei der Variante a) die Abtrennung des Antigen-Antikörper-Komplexes mit einem zweiten, gegen die Antikörper affinen Antikörper erfolgt.

5. Verwendung nach einem oder mehreren der vorhergehenden Ansprüche, dadurch gekennzeichnet, daß bei der Variante a) eine radioaktive Markierung eingesetzt wird.

6. Verwendung nach einem oder mehreren der vorhergehenden Ansprüche, dadurch gekennzeichnet, daß die Markierung mit Jod 125 erfolgt.

7. Verwendung nach einem oder mehreren der vorhergehenden Ansprüche, dadurch gekennzeichnet, daß bei der Variante b) ein Enzym zur Markierung dient.

8. Verwendung nach einem oder mehreren der vorhergehenden Ansprüche, dadurch gekennzeichnet, daß die Markierung im Komplex bestimmt wird.

**Revendications pour les Etats Contractants: BE, CH, DE, FR, GB, IT, LI, LU, NL, SE**

1. Antisérum hautement spécifique contre les deux antigènes peptide procollagène Col 1 (type III) et peptide procollagène (type III), pouvant être obtenu par immunisation d'animaux expérimentaux avec le peptide procrollagène Col 1 (type III).

2. Procédé pour la production d'un antisérum hautement spécifique contre les deux antigènes peptide procollagène Col 1 (type III) et peptide procollagène (type III), caractérisé en ce que l'on immunise des animaux expérimentaux avec le peptide procollagène Col 1 (type III) et on recueille leur sérum.

3. Utilisation des anticorps de l'antisérum selon la revendication 1, pour la détermination immunologique simultanée des deux antigènes peptide procollagène (type III) et peptide procollagène Col 1 (type III) caractérisée en ce que soit

a) on met en réaction une quantité déterminée dudit antisérum hautement spécifique, en excès par rapport à l'échantillon examiné, avec l'échantillon contenant lesdits antigènes et une quantité déterminée de l'un des deux antigènes sous forme marquée, on sépare le complexe antigène-anticorps formé et on détermine la quantité du marquage dans le complexe et/ou le surnageant, soit

b) on fait réagir avec l'échantillon à examiner une certaine quantité dudit antisérum, en excès par rapport à l'échantillon à examiner, on fixe avec un excès défini de l'un desdits antigènes qui sont liés à un support la quantité de l'anticorps n'ayant pas réagi et on la fait réagir avec un excès défini d'un second anticorps marqué, et ensuite on détermine la quantité du marquage du second anticorps lié et/ou libre.

4. Utilisation selon la revendication 3, caractérisée en ce que le marquage est un marquage radioactif, enzymatique ou fluorescent.

5. Utilisation selon une ou plusieurs des revendications précédentes, caractérisée en ce que, dans la variante a), on effectue la séparation du complexe antigène-anticorps à l'aide d'un second anticorps à affinité envers les anticorps.

6. Utilisation selon une ou plusieurs des revendications précédentes, caractérisée en ce que, dans la variante a), on utilise un marquage radioactif.

7. Utilisation selon une ou plusieurs des revendications précédentes, caractérisée en ce que le marquage est effectué avec de l'iode 125.

8. Utilisation selon une ou plusieurs des revendications précédentes, carctérisée en ce que, dans la variante b), on utilise une enzyme pour le marquage.

9. Utilisation selon une ou plusieurs des revendications précédentes, caractérisée en ce que l'on détermine le marquage dans le complexe.

**Revendications pour l'Etat Contractant: At**

1. Procédé pour la production d'un antisérum hautement spécifique contre les deux antigènes peptide procollagène Col. 1 (type III) et peptide procollagène (type III), caractérisé en ce que l'on immunise des animaux expérimentaux avec le peptide procollagène Col 1 (type III) et on recueille leur sérum.

2. Utilisation des anticorps de l'antisérum selon la revendication 1, pour la détermination immunologique simultanée des deux antigènes peptide procollagène (type III) et peptide procollagène Col. (type III), caractérisée en ce que soit

a) on met en réaction une quantité déterminée dudit antisérum hautement spécifique, en excès par rapport à l'échantillon examiné, avec l'échantillon contenant lesdits antigènes et une quantité déterminée de l'un des deux antigènes sous forme marquée, on sépare le complexe antigène-anticorps formé et on détermine la quantité du marquage dans le complexe et/ou le surnageant, soit

b) on fait réagir avec l'échantillon à examiner une certaine quantité dudit antisérum, en excès par rapport à l'échantillon à examiner, on fixe avec un excès défini de l'un desdits antigènes qui sont liés à un support la quantité de l'anticorps n'ayant par réagi et on la fait réagir avec un excès défini d'un second anticorps marqué, et ensuite on détermine la quantité du marquage du second anticorps lié et/ou libre.

3. Utilisation selon la revendication 2, caractérisée en ce que le marquage est un marquage radioactif, enzymatique ou fluorescent.

4. Utilisation selon une ou plusieurs des revendications précédentes, caractérisée en ce que, dans la variante a), on effectue la séparation du complexe antigène-anticorps à l'aide d'un second anticorps à affinité envers les anticorps.

5. Utilisation selon une ou plusieurs des revendications précédentes, caractérisée en ce que, dans la variante a), on utilise un marquage radioactif.

6. Utilisation selon une ou plusieurs des revendications précédentes, caractérisée en ce que le marquage est effectué avec de l'iode 125.

7. Utilisation selon une ou plusieurs des revendications précédentes, caractérisée en ce, dans la variante b), ou utilise une enzyme pour le marquage.

8. Utilisation selon une ou plusieurs des revendications précédentes, caractérisée en ce que l'on détermine le marquage dans le complexe.

**Claims for the Contracting States: BE, CH, DE, FR, GB, IT, LI, LU, NL, SE**

1. A highly specific antiserum against the two antigens procollagen peptide col 1 (type III) and procollagen peptide (type III), obtainable by immunization of experimental animals with procollagen peptide col 1 (type III).

2. A process for preparing a highly specific antiserum against the two antigens procollagen peptide col 1 (type III) and procollagen peptide (type III), which comprises immunizing experimental animals with procollagen peptide col 1 (type III) and obtaining their serum.

3. The use of the antibodies of the antiserum as claimed in claim 1 for the immunologic determination of the two antigens procollagen peptide (type III) and procollagen peptide col 1 (type III) together, wherein either

a) a specified amount, which is in excess relative to the sample to be investigated, of the said highly specific antiserum is reacted with the sample containing the said antigens and with a specified amount of one of the two antigens in labeled form, the antigen-antibody complex formed is separated off and the amount of labeling in the complex and/or supernatant is determined, or

b) a specified amount, which is in excess relative to the sample to be investigated, of the said antiserum is reacted with the sample to be investigated, the unreacted amount of the antibodies is immobilized with a defined excess of one of the said antigens which are bound to a carrier, and is reacted with a defined excess of a second labeled antibody, and then the amount of labeling in the bound and/or free second antibody is determined.

4. The use as claimed in claim 3, wherein the labeling is a radioactive, enzyme or fluorescence labeling.

5. The use as claimed in one or more of the preceding claims, wherein, in variant a), the separation off of the antigen-antibody complex is carried out with a second antibody having affinity for the antibodies.

6. The use as claimed in one or more of the preceding claims, wherein, in variant a), radioactive labeling is employed.

7. The use as claimed in one or more of the preceding claims, wherein the labeling is carried out with iodine-125.

8. The use as claimed in one or more of the preceding claims, wherein, in variant b), an enzyme is used for labeling.

9. The use as claimed in one or more of the preceding claims, wherein the labeling in the complex is determined.

**Claims for the Contracting State: AT**

1. A process for preparing a highly specific antiserum against the two antigens procollagen peptide col 1 (type III) and procollagen peptide (type III), which comprises immunizing experimental animals with procollagen peptide col 1 (type III) and obtaining their serum.

2. The use of the antibodies of the antiserum as claimed in claim 1 for the immunologic determination of the two antigens procollagen peptide (type III) and procollagen peptide col 1 (type III) together, wherein either

a) a specified amount, which is in excess relative to the sample to be investigated, of the said highly specific antiserum is reacted with the sample containing the said antigens and with a specified amount of one of the two antigens in labeled form, the antigen-antibody complex formed is separated off and the amount of labeling in the complex and/or supernatant is determined, or

b) a specified amount, which is in excess relative to the sample to be investigated, of the said antiserum is reacted with the sample to be investigated, the unreacted amount of the antibodies is immobilized with a defined excess of one of the said antigens which are bound to a carrier, and is reacted with a defined excess of a second labeled antibody, and then the amount of labeling in the bound and/or free second antibody is determined.

3. The use as claimed in claim 2, wherein the labeling is a radioactive, enzyme or fluorescence labeling.

4. The use as claimed in one or more of the preceding claims, wherein, in variant a), the separation off of the antigen-antibody complex is carried out with a second antibody having affinity for the antibodies.

5. The use as claimed in one or more of the preceding claims, wherein, in variant a), radioactive labeling is employed.

6. The use as claimed in one or more of the preceding claims, wherein the labeling is carried out with iodine-125.

7. The use as claimed in one or more of the preceding claims, wherein, in variant b), an enzyme is used for labeling.

8. The use as claimed in one or more of the preceding claims, wherein the labeling in the complex is determined.